# EUROPEAN PATENT SPECIFICATION

(11) **EP 4 008 288 B1**
(45) Date of publication and mention of the grant of the patent: **22.11.2023**
(21) Application number: 21212982.9
(22) Date of filing: 07.12.2021
(51) Int. Cl.: A61B 50/13, H02J 7/00, H02J 9/00, A61B 50/18

(54) **MEDICAL CART PROVIDING DATA CONTINUITY**
MEDIZINISCHER WAGEN MIT BEREITSTELLUNG VON DATENKONTINUITÄT
CHARIOT MÉDICAL FOURNISSANT UNE CONTINUITÉ DE DONNÉES

(30) Priority: 07.12.2020 US 202017113905
(43) Date of publication of application: 08.06.2022
(73) Proprietor: Capsa Solutions LLC, Portland OR 97230 (US)
(72) Inventor: PHILLIPS, Justin, Troutdale (US); ST. PIERRE, John, Portland (US); JONES, Aubrey, Estacada (US)
(74) Representative: Wynne-Jones IP Limited

(56) References cited:
- US-A1- 2015 223 891
- US-A1- 2017 141 597
- US-A1- 2020 206 400
- US-B1- 9 680 333

## Description

### CROSS-REFERENCE TO RELATED APPLICATIONS

This application is a continuation in part of U.S. Application No. 16/919,557 filed July 2, 2020, which claims the benefit of U.S. Provisional Patent Application No. 62/870,088 filed July 3, 2019.

### TECHNICAL FIELD

Exemplary embodiments relate generally to medical carts with data continuity.

### BACKGROUND AND SUMMARY OF THE INVENTION

Carts are often used in medical facilities for a variety of tasks. Such carts may be used, for example without limitation, to store and dispense medications, to input, store, and display electronic medical record information, to provide work stations and platforms, to store medical equipment, and the like. Such carts generally have a number of systems and components which require electrical power. Electrical power is often provided by way of one or more batteries. Sometimes, a cart has a first, external battery which is capable of being removed for charging, replacement, maintenance, and the like. In such cases, the cart may have a second, internal battery and/or connection to an external power source (e.g., plug) to facilitate the ongoing supply of electrical power. In some cases, it may be desirable to know in advance of the removal of the first battery to prevent power interruption, voltage spikes, or high in-rush current situations, all of which may result in damage or otherwise undesirable behavior to the end user. Therefore, in some situations it may be desirable to detect imminent battery removal. US9680333 discloses a power system for a medical cart. The power system includes a power supply, a direct current power bus electrically coupled to the power supply, and an electrical output power module electrically coupled to the direct current power bus. The power supply is positioned within the base of the medical cart and outputs direct current electrical power. The electrical output power module is within the work platform of the medical cart at the other end of the support. US2017/141597 discloses a mobile cart having a platform mounted above a wheeled based and at least one battery docking unit for the detachable mounting of a battery. A programmable control unit is associated with the at least one battery docking unit having a power output terminal at which a voltage is presented as the battery discharges.

The invention relates to a mobile medical cart providing data continuity as defined in independent claims 1 and 15. The invention further relates to a method of providing data continuity for a mobile medical cart as defined in independent claim 11. Further embodiments are specified in the dependent claims.

Certain systems and methods for detecting imminent battery removal are provided. In an exemplary embodiment, a cart may comprise a holder pivotably mounted thereto and configured to receive a battery. The holder may comprise battery terminals which facilitate electrical connection between the battery and a power bus while the battery is installed within the holder. The holder may rotate between a secured position and an unsecured position. While not in the unsecured position, the battery may be physically blocked from removal by way of an obstruction. A sensor may be positioned to detect movement of the holder from the secured position. The sensor may alert the power bus, which may upon such movement, disconnect from the battery and begin drawing upon a second battery or another power source as needed. However, the battery may remain electrically connected to the power bus during some or all of the time that the holder is moved between the secured and unsecured position. In exemplary embodiments, the battery remains electrically connected to the power bus by way of one or more battery terminals installed in the bottom of the holder such that the power bus is capable of drawing upon the battery, even when the holder is located in the unsecured position. The time it takes to move the holder from the secured position to the unsecured position may be long enough for the power bus to switch over to the second battery. In this way, the adverse effects associated with sudden disconnection from a power supply may be avoided.

In other exemplary embodiments, the first battery may be configured to fit within a holder. The holder may comprise an obstruction which permits the removal of the first battery unless and until the first battery is rotated away from the holder. A switch may be provided within the holder for detecting the presence of the first battery. A connector for the battery terminals of the first battery may pivot to maintain an electrical connection with the first battery while the first battery is rotated. A protrusion within the holder may fit within a cavity of the first battery, but be configured to permit the aforementioned rotation. The cart may switch to the second battery when the first battery is moved and the switch deactivated.

In some cases, there is no way to know that the first battery will be removed. For example, the cart may collide with a wall, personnel, another cart, or the like and the first battery may become dislodged. Still other problems, such as malfunctions, may result in power interruption where no advance notice can be given. Therefore, in some situations it may be desirable to switch power supplies automatically.

A cart configured to source power from a back-up power supply automatically upon determination of an unanticipated loss of power event from the first battery is provided, along with methods for operating the same. The cart may comprise a holder configured to receive a first, removeable battery. The holder may comprise battery terminals which facilitate electrical connection between the first battery and a power bus while the first battery is installed within the holder. The cart may comprise a second, back-up battery. A power bus may be electrically connected to the first, removeable battery and the second, back-up battery. The unanticipated loss of power event may be determined by certain electrical changes occurring after the first battery is unexpectedly removed, such as but not limited to, voltage spikes, voltage drops, current spike, current drops, capacity drops, some combination thereof, or the like. The unanticipated loss of power may be indicated to the user such that the user may go about restoring the first battery power, such as by replacing the first battery with another battery, recharging the first battery, supplying alternative power (e.g., plugging the cart into a wall outlet), or the like. Power sourcing may be of just the first battery or just the second battery, or some combination thereof. Such automatic changing of supplied power may be accomplished without the need for any detection of user interaction, especially preemptive indication of user interaction.

In some cases, users choose to remove the first battery from the cart before it is completely discharged. For example, the user might remove the first battery at the end of their shift for recharging, even though the battery is still at 50 percent capacity. Similarly, the cart may periodically run on a second battery for a period of time while a first battery is swapped without completely discharging. This may result in battery memory effects, which may result in the battery having reduced capacity and other issues over time. Such memory affects may apply to one or both of the first battery and the second battery. Therefore, in some situations it may be desirable to change the power supply automatically after complete, or near complete, discharge of the first and/or second battery.

A medical cart which changes power supply automatically upon complete, or near complete, discharge is provided. A cart may comprise a holder configured to receive a first, removeable battery. The holder may comprise battery terminals which facilitate electrical connection between the first battery and a power bus while the first battery is installed within the holder. The cart may comprise a second, back-up battery. A power bus may be electrically connected to the first, removeable battery and the second, back-up battery. The cart may be configured to draw upon the first battery until the first battery is exhausted. Once exhausted, the cart may be configured to automatically source power from the second, back-up battery. Once the back-up battery is exhausted, the cart may be configured to automatically source power again from the first battery. In this way, the cart may be configured to continuously move between fully exhausting the first battery and fully exhausting the second battery to preserve continuity of data in a way which also reduces or eliminates battery memory effects. Indication may be provided at the cart that the external battery is exhausted such that the external battery may be recharged or swapped with another battery or an alternative power source (e.g., outlet power) may be utilized. Power sourcing may be of just the first battery or just the second battery, or some combination thereof. Such automatic changing of supplied power may be accomplished without the need for any detection of user interaction, especially preemptive indication of user interaction.

In exemplary embodiments, a voltage detection circuit (VDC) may be provided which is in electrical connection with the removable battery and/or an alternative power source. Upon detection of a voltage decrease at the VDC, a microcontroller may command a switching device to switch power sourcing from the removable battery to the alternative power source. Upon detection of a voltage increase at the VDC, the microcontroller may determine if a valid charge state exists. If so, the microcontroller may command the switching device to switch power sourcing from the alterative power source to the removable battery. If not, the microcontroller may command the switching device to continue sourcing power from the alternative power source. The switching device may be electrically interposed between the removable battery and the alternative power source and various system boards for the cart.

Further features and advantages of the systems and methods disclosed herein, as well as the structure and operation of various aspects of the present disclosure, are described in detail below with reference to the accompanying figures.

### BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical or equivalent features, and wherein:
**FIGURE 1A** is a side view of an exemplary holder for a cart with an exemplary battery in a secured position in accordance with the present invention;
**FIGURE 1B** is a side view of the holder of figure 1A in a partially unsecured position;
**FIGURE 1C** is a side view of the holder of figure 1A in a fully unsecured position;
**FIGURE 1D** is a side view of the battery of figure 1A removed from the holder;
**FIGURE 2A** is a side view of an exemplary cart with the holder of figure 1A in the secured position;
**FIGURE 2B** is a side view of the cart of figure 2A with the holder in the unsecured position;
**FIGURE 3** is a flow chart with exemplary logic for use with the cart of figures 1A-2B;
**FIGURE 4** is a flow chart with exemplary logic for use with the cart of figures 1A-2B;
**FIGURE 5** is a side view of another exemplary embodiment of the cart with another exemplary battery removal detection system;
**FIGURE 6A** is a detailed side view of the battery removal detection system of figure 5 with the removeable battery in a secured position;
**FIGURE 6B** is a detailed side view of the battery removal detection system of figure 5 with the removeable battery in a partially unsecured position;
**FIGURE 6C** is a detailed side view of the battery removal detection system of figure 5 with the removeable battery in a fully unsecured position;
**FIGURE 6D** is a detailed side view of the battery removal detection system of figure 5 with the removeable battery removed from the holder;
**FIGURE 7** is a flow chart with exemplary logic for operating the cart of figures 5-6D;
**FIGURE 8** is a flow chart with exemplary logic for operating the cart of figures 5-6D;
**FIGURE 9** is a side view of another exemplary cart with an exemplary automatic power sourcing system;
**FIGURE 10** is a flow chart with other exemplary logic for operating the cart of figures 5-6D and 9;
**FIGURE 11** is a flow chart with other exemplary logic for operating the cart of figures 5-6D and 9;
**FIGURE 12** is a front perspective view of an exemplary cart in accordance with the present invention;
**FIGURE 13** is a rear perspective view of the cart of figure 12;
**FIGURE 14** is an exemplary electrical system diagram for the cart of figure 12;
**FIGURE 15** is a flow chart with other exemplary logic for operating the cart of figure 12;
**FIGURE 16** is a flow chart with other exemplary logic for operating the cart of figure 12;
**FIGURE 17A** is a plan view of an exemplary electrical schematic for use with the cart of figure 12; and
**FIGURE 17B** is a plan view of further components of the exemplary electrical schematic of figure 17A.

### DETAILED DESCRIPTION OF EXEMPLARY EMBODIMENT(S)

Various embodiments of the present invention will now be described in detail with reference to the accompanying drawings. In the following description, specific details such as detailed configuration and components are merely provided to assist the overall understanding of these embodiments of the present invention. In addition, descriptions of well-known functions and constructions are omitted for clarity and conciseness.

Embodiments of the invention are described herein with reference to illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

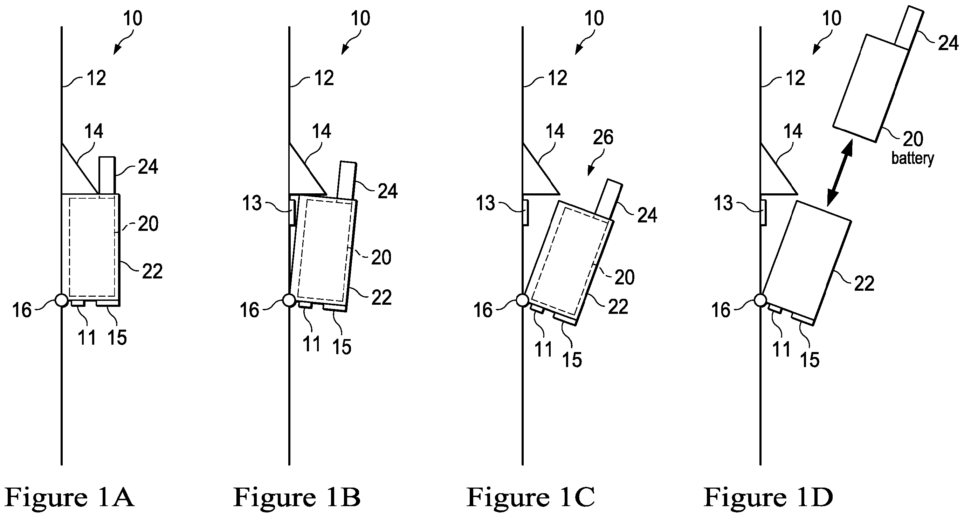

**FIGURES 1A-1D** are simplified diagrams of a portion of a medical cart 10 comprising a holder 22 configured for movement between a secured position (figure 1A), a partially unsecured position (figure 1B), and an unsecured position (figure 1C-1D) which permits removal or insertion of a battery 20. A pivot device 16 may be installed to a surface 12 of the medical cart 10. The pivot device 16 may be connected to the holder 22. The holder 22 may define a container or receptacle sized to accommodate the battery 20. The holder 22 may be configured to accommodate one of a number of interchangeable batteries 20 in exemplary embodiments. The pivot device 16 may comprise a hinge, bearing, or other rotational mechanism which permits the holder 22 to be rotated towards and away from the surface 12 of the cart 10.

The pivot device 16 may be configured to permit the holder 22, and thus the battery 20 when installed, to be moved between a secured position, whereby the holder 22 extends parallel to, and/or rests against, the surface 12, and an unsecured position whereby the holder 22 is positioned at an angle from the surface 12. The pivot device 16 may have a limited range of motion which limits the distance the holder 22 may be rotated away from the surface 12 so as to prevent the battery 20 from falling out when the holder 22 is in the unsecured position. Alternatively, or additionally, the holder 22 may comprise one or more mechanisms configured to temporarily secure the battery 20 such as, but not limited to, magnets, press fits, mating protrusions and recesses, notches, locks, some combination thereof, or the like.

An obstruction 14 may protrude from the surface 12. The obstruction 14 may be configured to prevent the battery 20 from being removed from, or inserted into, the holder 22 when the holder 22 is in the secured position and/or the partially unsecured position. The obstruction 14 may be configured to allow the battery 20 to be removed from, or inserted into, the holder 22 when the holder 22 is in the fully unsecured position. While in the fully unsecured position, a gap 26 may be formed between the obstruction 14 and the holder 22 such that the battery 20 may be removed from, or inserted into, the holder 22. The battery 20 may comprise a handle 24 which may facilitate removal and insertion of the battery 20 into or from the holder 22. Alternatively, or additionally, the same or a different handle 24 may be located on the holder 22 to facilitate rotational movement of the holder 22. While the obstruction 14 is illustrated as triangular in shape, those of skill in the art will recognize that any size or shape obstruction 14 is contemplated. A combination of obstructions 14 may be utilized in certain embodiments.

The holder 22 may comprise a first sensor 11. The first sensor 11 may be configured to detect whether the battery 20 is installed within the holder 22. The first sensor 11 may comprise a pressure sensor, weight sensor, proximity sensor, light sensor, switch, some combination thereof, or the like. In exemplary embodiments, the first sensor 11 may be located along a bottom surface of the holder 22 such that the battery 20 contacts the first sensor 11 upon insertion within the holder 22, though such is not required.

A second sensor 13 may be located at the surface 12. The second sensor 13 may be configured to detect whether the holder 22 is in the secured and/or unsecured position. The second sensor 13 may comprise a pressure sensor, weight sensor, proximity sensor, light sensor, switch, some combination thereof, or the like. In exemplary embodiments, the second sensor 13 may be located below the obstruction 14 such that the holder 22 contacts the second sensor 13 upon placement in the secured position against or parallel to the surface 12, though such is not required.

Alternatively, or additionally, the second sensor 13 may comprise a movement-based sensor such as, but not limited to, an accelerometer, angle sensor, some combination thereof, or the like. In such embodiments, the second sensor 13 may be configured to detect movement of the holder 22 and/or the rotational device 16.

To remove or insert the battery 20, a user may pivot the holder 22 away from the surface 12 by pulling on the handle 24 to create the gap 26 such that the battery 20 may clear the obstruction 14. The pivot device 16, the holder 22, the surface 12, and/or the obstruction 14 may be configured to require a predetermined amount or force to initially move the holder 22 from the secured position. Once rotated to the unsecured position, the battery 20 may be removed by pulling the handle 24 and lifting the battery 20 out of the holder 22. Once rotated to the unsecured position, the battery 20 may be inserted by grasping the handle 24 and placing the battery 20 within the holder 22. The obstruction 14 may be sized, shaped, or otherwise configured to prevent removal or insertion of the battery 20 while the holder 22 is in the secured position and the partially unsecured position and permit removal or insertion of the battery 20 while the holder 22 is in the unsecured position.

The holder 22 may further comprise one or more battery terminals 15. The battery terminals 15 may comprise any device which facilitates electrical connection with the battery 20. In exemplary embodiments, the battery terminals 15 may be located along a bottom surface of the holder 22, though such is not required.

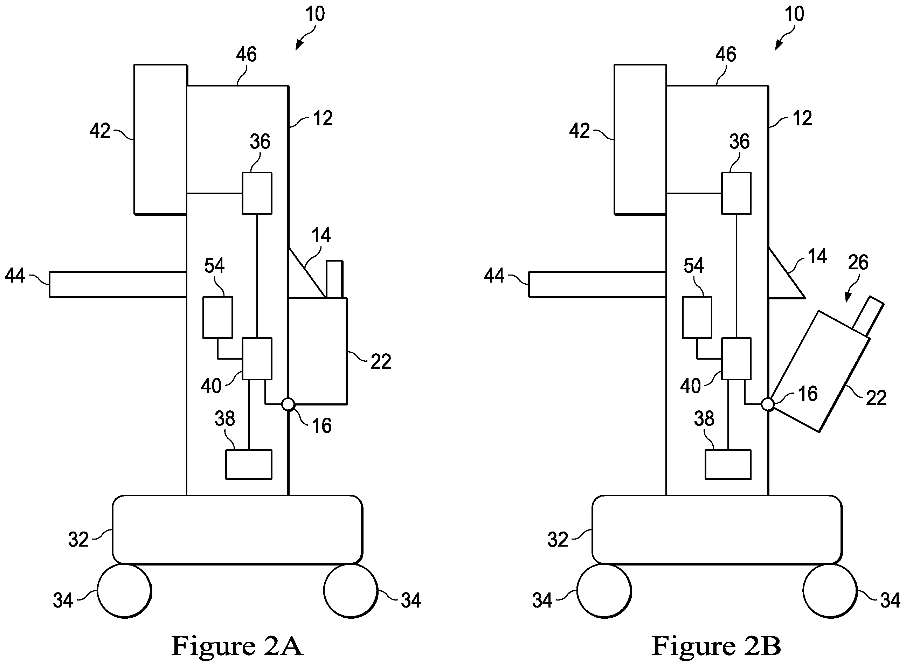

**FIGURES 2A-2B** illustrate an exemplary cart 10 with the holder 22 in the secured position (figure 2A) and the fully unsecured position (figure 2B). The cart 10 may be any kind of cart 10 comprising any number of features and/or components. The cart 10 may comprise a body 46, a base 32, wheels 34, and a work platform 44, to name a few examples. The cart 10 may further comprise one or more displays 42. In particular, the cart 10 may be a medical cart for use in hospitals, doctor's offices, skilled care facilities, nursing homes, and other medical care facilities. The cart 10 may be configured, for example without limitation, to store and dispense medications, to input, store, and display electronic medical record information, to provide work stations and platforms, to store medical equipment, some combination thereof, and the like. The illustrated cart 10 is shown merely for exemplary purposes and is not intended to be limiting. For example, without limitation, the body 46, base 32, wheels 34, and work platform 44 may be provided in any size and/or shape, in addition to other components, and/or may be excluded.

The cart 10 may comprise a number of electrical components 36 placed in electrical connection with the battery 20. In exemplary embodiments, such electrical connection may be made by way of a power bus 40 and/or one or more battery terminals 15. The electrical connection may be maintained while the holder 22 is rotated between the secured, the partially unsecured, and the fully unsecured positions. The electrical connection to the battery 20 may be lost only upon removal of the battery 20 from the holder 22. In exemplary embodiments, the battery 20 is capable of remaining electrically connected to the power bus 40 by way of one or more battery terminals 15 such that the power bus 40 is capable of drawing upon the battery 20, even when the holder 22 is located in the fully unsecured position.

The electrical components 36 may also be electrically connected to a second battery 38. Both the first battery 20 and the second battery 38 may be configured to operate the cart 10 and various functions and components thereof for the same or different periods of time. The battery 20 may be external, while the second battery 38 may be internal, though such is not required. In other exemplary embodiments, an external power source may be used in lieu of, or in addition to, the second battery 38, such as but not limited to, a plug for connecting to a wall outlet. The electrical components 36 may be electrically connected to the battery 20 and the second battery 38 by way of the power bus 40. One such electricity consuming component 36 may be the one or more displays 42, which may comprise an electronic display. Other such electricity consuming components 36 include, for example without limitation, computers, displays, touch screens, user interfaces, medical equipment, locking devices, motors, lift mechanisms, locking bins, lights, some combination thereof, or the like.

In exemplary embodiments, the power bus 40 may be in electrical connection with a controller 54. The controller 54 may be configured to control certain operations of the cart 10 shown and described herein.

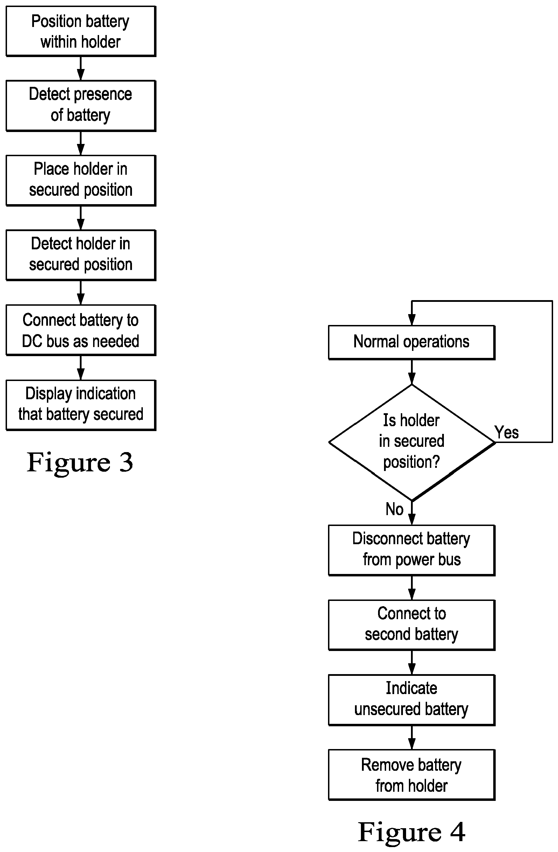

**FIGURE 3** is a flow chart with exemplary logic for installing the removable battery 20. The removable battery 20 may be secured within the holder 22. The presence of the battery 20 may be detected by the first sensor 11. In exemplary embodiments, the presence of the battery 20 may be indicated to the user, such as by display at the display 42, audible alert, notification, some combination thereof, or the like. The holder 22 may be placed in the secured position. Placement of the holder 22 in the secured position may be detected by the second sensor 13. In exemplary embodiments, placement of the holder 22 in the secured position may be indicated to the user, such as by display at the display 42, audible alert, notification, some combination thereof, or the like. In exemplary embodiments, the cart 10 may be configured to draw upon the removeable battery 20 as needed or programmed when the holder 22 is placed in the secured position. For example, the cart 10 may be configured to prevent drawing power from the battery 20 until the holder 22 is placed in the secured position and thus blocked from removal by the obstruction 14. Operations described herein may be performed, at least in part, by instruction from the controller 54.

**FIGURE 4** is a flow chart with exemplary logic for removing the battery 20. The cart 10 may be configured to periodically or continually monitor for movement of the holder 22. Once it is determined that the holder 22 is out of the secured position, such as in the partially and/or fully unsecured positions, the cart 10 may be configured to stop or continue not drawing power from the first battery 20. The cart 10 may be configured to begin utilizing the second battery 38 and/or an alternative power source as needed or otherwise programmed. Movement of the holder 22 from the secured position may be detected by the second sensor 13. Placement of the holder 22 in the partially or fully unsecured position may be indicated to the user, such as by display at the display 42, audible alert, notification, some combination thereof, or the like. Once in the fully unsecured position, the battery 20 may be removed from the holder 22. The time it takes to move the holder 22 from the secured position to the fully unsecured position may provide sufficient time for the cart 10 to begin sourcing power only from the second battery 38 and/or an alternative power source. Removal of the battery 20 from the holder 22 may be detected by the first sensor 11 and indicated to the user, such as by display at the display 42, audible alert, notification, some combination thereof, or the like.

For clarity, in exemplary embodiments, the potential for an electrical connection between the battery 20 and the power bus 40 may be maintained while the holder 22 is rotated. In this way, the power bus 40 may continue to draw upon the battery 20 while the holder 22 is rotated. The potential for an electrical connection may be terminated, in exemplary embodiments, only upon removal of the battery 20 from the holder 22. However, the cart 10 may be configured to cease drawing upon the battery 20 once it is determined, or at some point following, that the holder 22 is no longer in the secured position. In this way, adequate time is allotted for switchover to the second battery 38 and/or alternative power source such that constant electrical supply is maintained and the negative effects of interrupted power are not experienced.

Operations described herein may be performed, at least in part, by instruction from the controller 54.

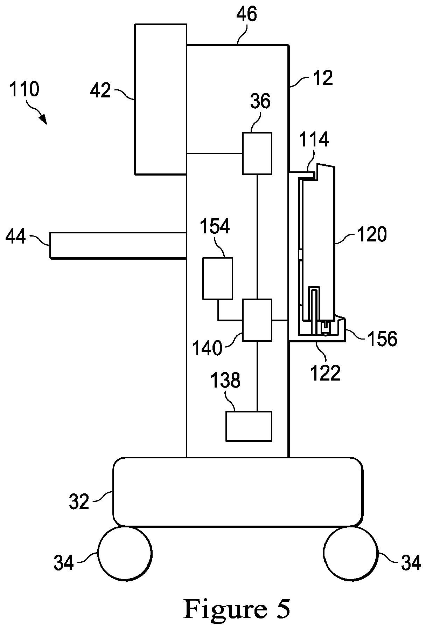

**FIGURE 5** illustrates another exemplary cart 110. A holder 122 may be mounted to the cart 110. The holder 122 may be configured to removably accept a first battery 120. The first battery 120 may be configured to power the cart 110 for a period of time. Such period of time may be at least a number of minutes, for example. The holder 122 may be configured to interchangeably receive one of a number of identical or similar such batteries 120. The holder 122 may comprise one or more obstructions 114 configured to prevent removal of a secured one of the removable batteries 120 without movement of the battery 120.

A power bus 140 may be electrically connected to the first battery 120 when installed at the holder 122. The power bus 140 may be electrically connected to a back-up power source 138. The back-up power source 138 may be configured to power the cart 110 for a period of time. Such period of time may be at least a number of minutes, for example. The back-up power source 138 may be permanently, or semi-permanently installed at the cart 110 such that it is not capable of being readily removed, such as without particular tools. The back-up power source 138 may comprise one or more batteries, generators, plug for wall outlet, some combination thereof, or the like.

In exemplary embodiments, the power bus 140 may be in electrical connection with a controller 154. The controller 154 may be configured to control certain operations of the cart 110 shown and described herein.

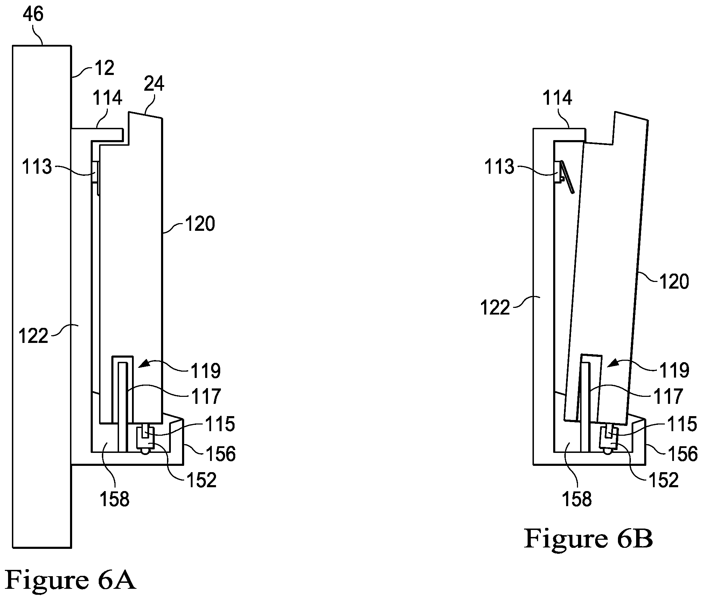

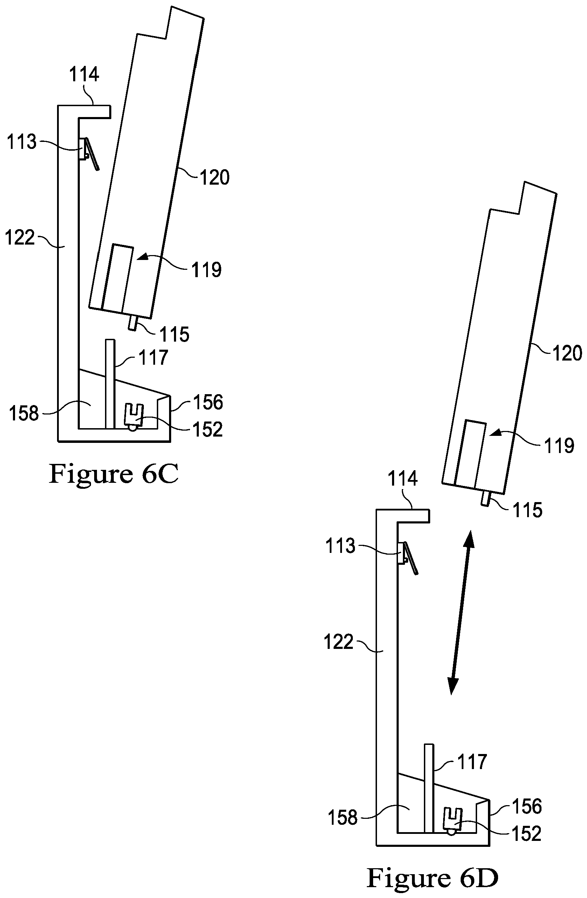

**FIGURES 6A-6D** illustrate detailed views of the holder 122, battery 120, and other components. The holder 122 may comprise a protrusion 117. The battery 120 may comprise a cavity 119 configured to receive the protrusion 117. Any shape, size, or type of cavity 119 and/or protrusion 117 may be utilized. Multiple protrusions 117 and cavities 119 may be utilized. In exemplary embodiments, the cavity 119 may be complementary to the protrusion 117, however, sufficient clearance may be provided to permit rotation or other movement of the battery 120 between the secured, partially unsecured, and fully unsecured positions such that the battery 120 may be positioned to clear the obstruction for removal from, or insertion into, the holder 122. The cavity 119 and protrusion 117 may be configured to mate to guide the battery 120 into the holder 122 and maintain its position within the holder 122 until manually removed by the user. The holder 112 may comprise a front lip 156 and/or side walls 158 configured to assist with securing the battery 120 within the holder 122 during such removal and insertion.

A switch 113 may be provided within the holder 122. The switch 113 may be configured to detect the battery 120 when it is secured within the holder 122. For example, without limitation, the switch 113 may be deactivated when the battery 120 is in the partially and/or fully unsecured positions and may be activated when the battery 120 is in the fully secured position. Other sensors may be used in conjunction with, or alternatively to, the switch 113.

A connector 152 may be provided within the holder 122. One or more terminals 115 may be provided at the battery 120. When the battery 120 is in the secured, partially unsecured, and/or fully unsecured positions, the connector 152 may be configured to maintain a potential electrical connection with the terminals 115 on the battery 120. In exemplary embodiments, without limitation, the connector 152 may be provided with a swivel, spring, extension device, some combination thereof, or the like to maintain the potential for such a connection. The cavity 119 and the protrusion 117 may be configured to align the connector 152 with the terminals 115 when the battery 120 is installed and removed from the holder 122. The obstruction 114 may be configured to prevent manual removal of the battery 120, and thus breaking of the connection between the connector 152 and the terminals 115, prior to the battery 120 being placed in the fully unsecured position.

The fully secured position may include placing the battery 120 vertically within the holder 122, though any position may be utilized. The partially and fully unsecured positions may comprise rotating the battery 120 away from the body 46 at an increasing angle.

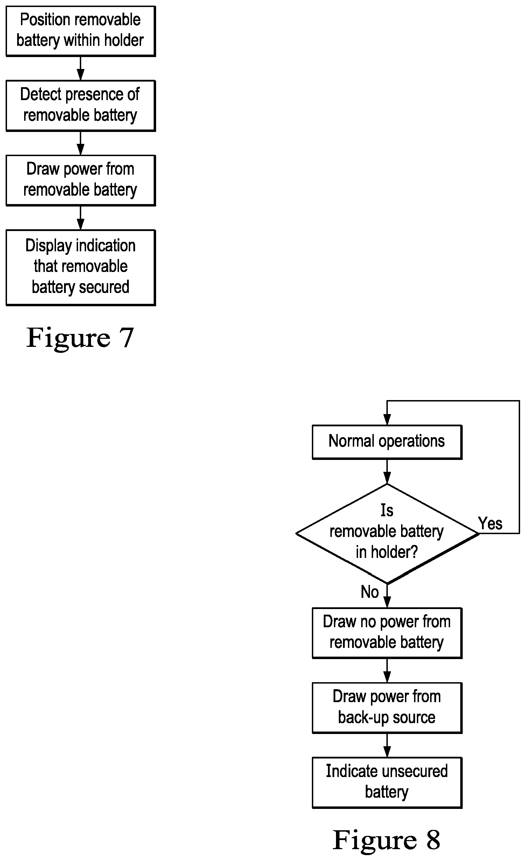

**FIGURE 7** and **FIGURE 8** illustrates exemplary logic for operating the cart 110. The presence or non-presence of the removeable battery 120 within the holder 122 may be determined by the switch 113. The power bus 140 may begin and/or continue drawing power from the battery 120, as needed, upon the determination of presence of the battery 120. The power bus 140 may cease and/or not initiate drawings power from the battery 120 upon determination of non-presence of the battery 120. The power bus 140 may begin and/or continue drawing power from the back-up power source 138, as needed, upon the determination of non-presence of the battery 120. The power bus 140 may cease and/or not initiate drawings power from the back-up power source 138 upon determination of presence of the battery 120.

The current power sourcing status, such as whether the cart 110 is drawing power from one or both of the battery 120 and/or back-up power source 138, may be indicated at the cart 110. Such indication may be made by audible signal, visual signal, display at the display 42, some combination thereof, or the like. The amount of power being drawn from each source may be indicated along with other information such as, but not limited to, estimated power time remaining, capacity, voltage, current, run time, charging/discharging status, cycle count, some combination thereof, or the like.

Operations described herein may be performed, at least in part, by instruction from the controller 154.

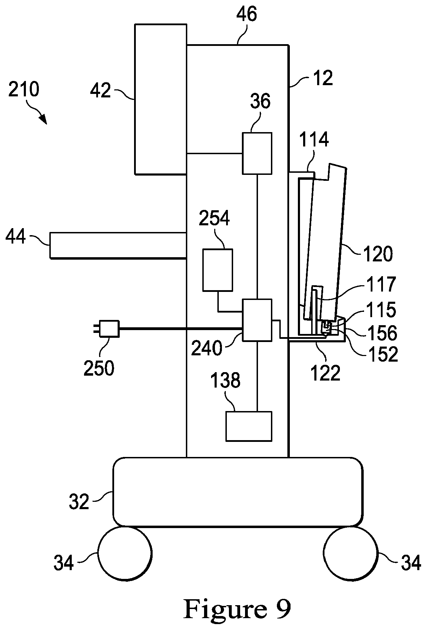

**FIGURE 9** illustrates another exemplary embodiment of the cart 210. A plug 250 may be provided for connection to wall outlet or other utility power. The plug 250 may be in electrical connection with a power bus 240. Operations described herein may be performed, at least in part, by instruction from the controller 254. The controller 254 may be configured to control certain operations of the cart 110 shown and described herein.

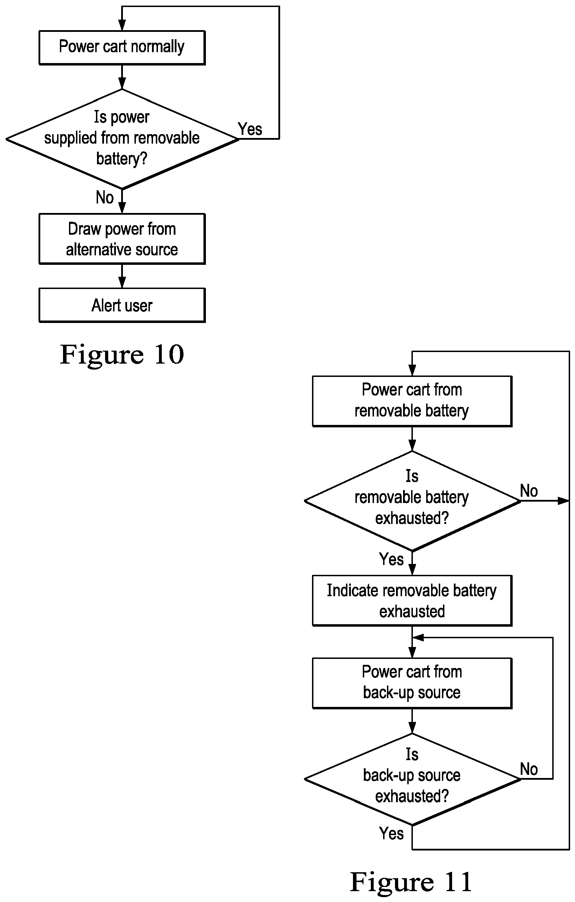

**FIGURE 10** illustrates exemplary logic for operating the cart 210. The cart 210 may be operated normally. Such normal operation may comprise drawing power from the removeable battery 120, utility power (via the plug 250), and/or the backup battery 138. Normal operations may continue until an unexpected loss of power event is determined such as, but not limited to, removal, dislodgement, disconnection, or the like of the battery 120. Upon determination of the unexpected loss of power event, the power bus 240 may be configured to automatically begin drawing power from an alternative power source, such as but not limited to, the back-up battery 138, utility power (via the plug 250), some combination thereof, or the like. Drawing of power from the alternative power source may occur automatically and may occur only after determination of the unexpected loss of power event. In exemplary embodiments, the unexpected loss of power event may be determined by a voltage drop, spike, current drop, spike, capacity loss, some combination thereof, or the like. The user may be alerted to the unexpected loss of power event by audible signal, visual signal, display at the display 42, some combination thereof, or the like. Operations described herein may be performed, at least in part, by instruction from the controller 254.

**FIGURE 11** illustrates other exemplary logic for operating the cart 210. The cart 210 may be configured to draw power from the removeable battery 120 until capacity of the removeable battery 120 is exhausted. After exhaustion of the removeable battery 120, the power bus 240 may automatically draw power from an alternative power source, such as but not limited to, the back-up battery 138, utility power supply (via the plug 250), some combination thereof, or the like. Drawing of power from the alternative power source may occur automatically and may occur only after exhaustion of the battery 120 is completed. In exemplary embodiments, the loss of capacity may be determined by a voltage drop, spike, current drop, spike, capacity loss, some combination thereof, or the like.

The power bus 240 may automatically power the cart 210 from the alternative power source until the alternative power source is exhausted. Following such exhaustion of the alternative power source, the power bus 240 may draw power from the removeable battery 120. Alternatively, or additionally, the power bus 240 may automatically power the cart 210 from the alternative power source until capacity of the removeable battery 120 is restored, such as but not limited to a non-zero capacity, or capacity above the threshold.

The user may be alerted to the loss of capacity and/or change in power sourcing by audible signal, visual signal, display at the display 42, some combination thereof, or the like. The user may alternatively, or additionally, be provided one or more warning alerts indicating capacity below a certain threshold (e.g., 10%).

The drawing of power from a particular power source (e.g., battery 120, back-up battery 138, plug 250) as described herein may be realized exclusively or in combination with drawing power from other power sources (e.g., battery 120, back-up battery 138, plug 250).

Operations described herein may be performed, at least in part, by instruction from the controller 254.

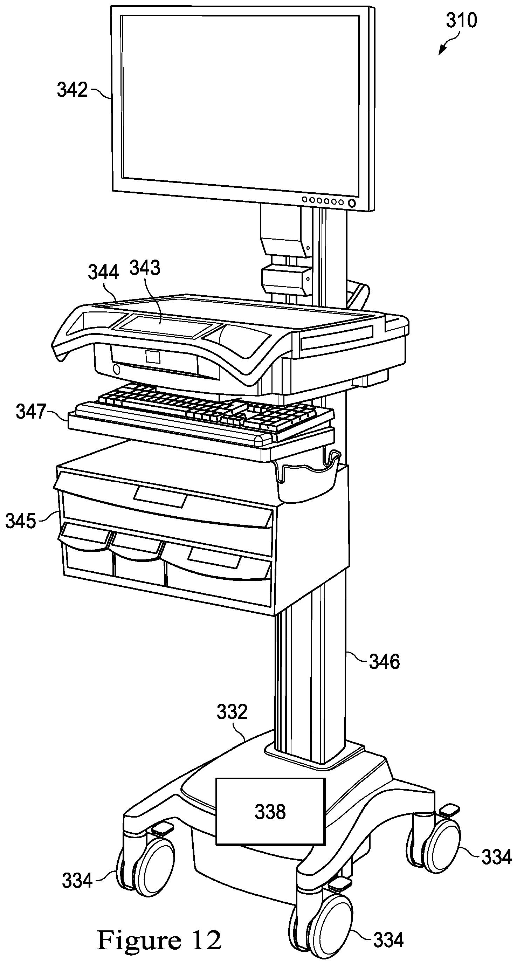

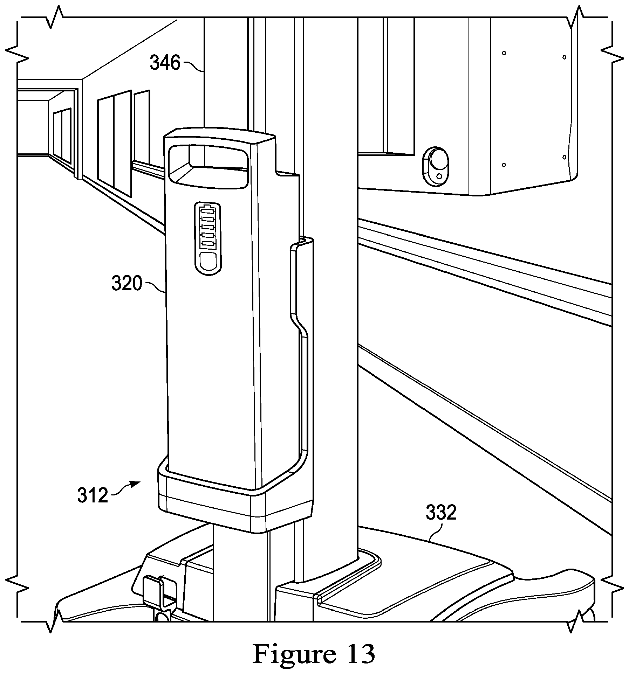

**FIGURE 12** and **FIGURE 13** illustrate another exemplary embodiment of the cart 310. Similar elements may be numbered similarly but increased by 100 (e.g., 210 to 310). A body 346 may extend vertically from a base 332 comprising one or more wheels 334. The wheels 334 may be provided in the form of casters, though other types and kinds of wheels 334 may be utilized. One or more of the wheels 334 may be powered by respective motors to assist in moving the cart 310. The body 346 may comprise one or more rigid members. The body 346 may comprise a hollow, cuboid shape though any shape of body 346 may be utilized. An alternative power source 338 may be located in the base 332 so as to provide a stable foundation for the cart 310, though the alternative power source 338 may be located elsewhere.

A removable battery 320 may be selectively engaged with a holder 312 located at the body 346. In exemplary embodiments, the holder 312 may be attached to a rear portion of the body 346 above the base 332, though other locations, including but not limited to providing the holder 312 at the base 332 may be utilized. Said holder 312 may be configured to provide a snap-fit, friction-fit, or the like for said removable battery 320.

A display 342 may be mounted to an upper portion of said body 346. The display 342 may be configured to provide status information regarding the cart 310, patient information, combinations thereof, or the like.

One or more storage devices 345 may be provided along the body 346. The storage devices 345 may be located below the display 342. Any number, size, or type of storage devices 345 or other such as but not limited to, drawers, cubbies, shelves, cabinets, or the like may be utilized. In exemplary embodiments, some or all of the storage devices 345 may be individually lockable by way of an electronically controlled locking system.

A tray 347 may be provided along the body 346. The tray 347 may be located above or between the storage devices 345. The tray 347, in exemplary embodiments, may be configured for selective movement out and away from the body 346. The tray 347 may be configured to accommodate a keyboard.

A work platform 344 may be mounted to the body 346 at a position between said base 332 and said display 342, though any location may be utilized. The work platform 344 may comprise a flat surface, handles, and a control system 343. The control system 343 may comprise one or more computerized systems, touch screens, buttons, combinations thereof, or the like. The control system 343 may be configured to control various functions of the cart 310, including but not limited to, the height of the work platform 344, the content displayed at the display 342, content of the storage devices 345, locking mechanisms for the storage devices 345, status information for the cart 310, and the like. The status information for the cart 310 may include, for example without limitation, information regarding whether power is being sourced from said alternative power source 338 or a removable battery 320, amount of power remaining at said alternative power source 338 or said removable battery 320, charging status, discharge status, combinations thereof, or the like.

The holder 312 may be the same or similar to those shown and/or described with regards to figures 1-11.

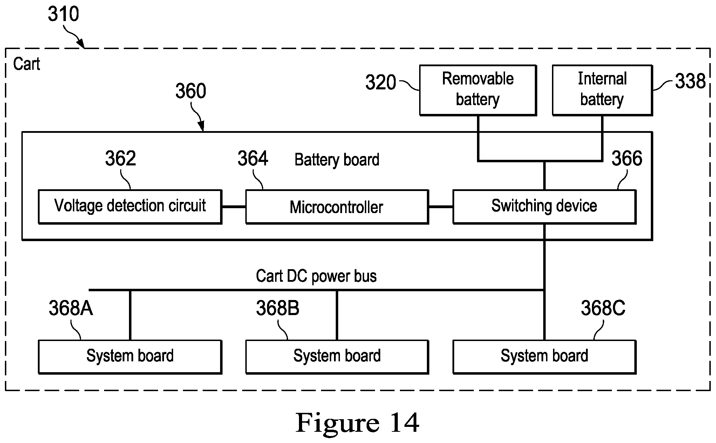

**FIGURE 14** illustrates an exemplary battery board 360 for the cart 310. The battery board 360 may be installed anywhere at the cart 310, though in exemplary embodiments the battery board 360 is located within the work platform 344. The battery board 360 may comprise a voltage detection circuit (VDC) 362, a microcontroller 364, and/or a switching device 366.

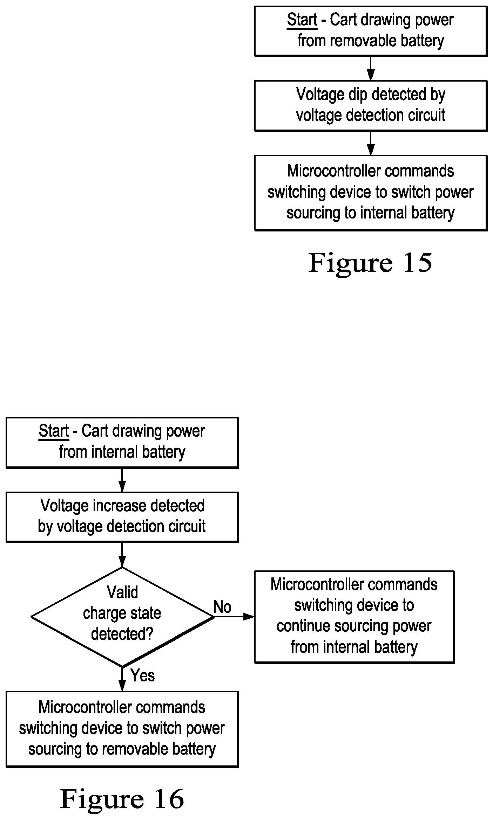

As provided in **FIGURE 15** and **FIGURE 16** the VDC 362 may be in electrical connection with the removable battery 320 and/or an alternative power source 338. Upon detection of a voltage decrease at the VDC 362, the microcontroller 364 may be configured to command the switching device 366 to switch power sourcing from the removable battery 320 to the alternative power source 338. The decrease may be a dip, gradual decrease, rapid decrease, drop off, combinations thereof, or the like. Upon detection of a voltage increase at the VDC 362, the microcontroller 364 may be configured to determine if a valid charge state exists. The increase may be a spike, gradual increase, rapid increase, jump, combinations thereof, or the like. If so, the microcontroller 364 may be configured to command the switching device 366 to switch power sourcing from the alterative power source 338 to the removable battery 320. If not, the microcontroller 364 may be configured to command the switching device 366 to continue sourcing power from the alternative power source 338. The switching device 366 may be electrically interposed between the removable battery 320 and the alternative power source 338 and various system boards for the cart 368A-C such that the battery board 360 may control power sourcing for such various cart 310 systems.

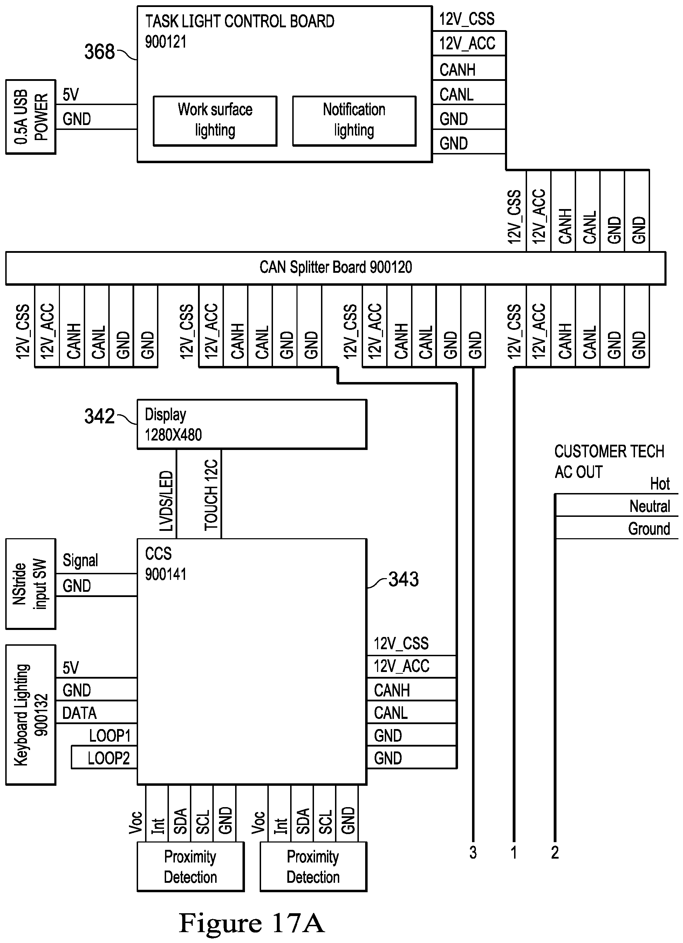

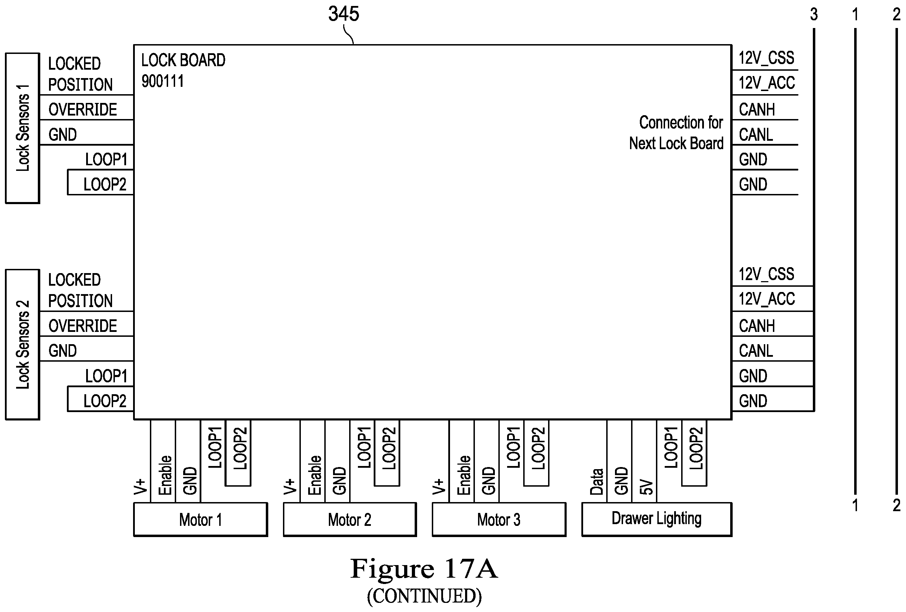

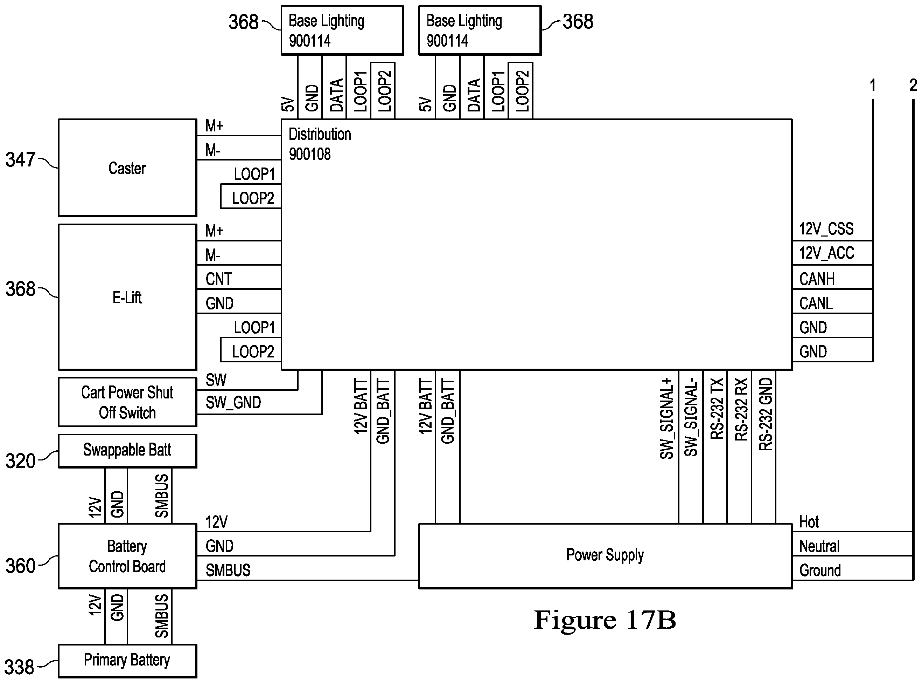

**FIGURE 17A** through **FIGURE 17B** illustrates an exemplary circuit diagram for the cart 310. Various cart 310 systems may be connected to the battery board 360 including, but not limited to, various system boards 368 for various functions of the cart 310 including but not limited to task lights, base lights, lifts for the work platform 344, displays 342, or other cart 310 components. The various system boards 368 may alternatively or additionally control, for example without limitation, the storage devices 345 and locking mechanisms for the same, the control system 343, the display 342, the wheels 334, combinations thereof, or the like.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention..

Certain operations described herein may be performed by one or more electronic devices. Each electronic device may comprise one or more processors, electronic storage devices, executable software instructions, and the like configured to perform the operations described herein. The electronic devices may be general purpose computers or specialized computing devices. The electronic devices may be personal computers, smartphones, tablets, databases, servers, or the like. The electronic connections described herein may be accomplished by wired or wireless means.

## Claims

1. A mobile medical cart (310) providing data continuity, said mobile medical cart comprising:
a base (332) comprising wheels (334) for moving said mobile medical cart about a ground surface;
a body (346) extending upward from said base;
a display (342) mounted to an upper portion of said body;
system boards (368) for controlling functions of said mobile medical cart;
a work platform (344) mounted to said body between said display and said wheeled base;
a control system (343) in electronic communication with said system boards and configured to receive user input and command changes to said functions of said mobile medical cart based upon said user input;
a holder (312) mounted to the body above said base and configured to removably receive a removable battery (320) comprising a terminal (115) in an interchangeable fashion with other removable batteries (320);
a connector (152) located at the holder and positioned to receive the terminal when said removable battery is positioned within the holder;
an alternative power source (338) comprising a back-up battery permanently installed within said mobile medical cart and configured to power said mobile medical cart for a period of time; and
a battery board (360) comprising a voltage detection circuit (362), a microcontroller (364), and a switching device (366), wherein said switching device is electrically interposed between said alternative power source, said connector of said holder, and each of said system boards, wherein said battery board is configured to:
automatically and electronically command, by way of said microcontroller, said switching device to begin sourcing power solely from said alternative power source following detection of a voltage decrease at said voltage detection circuit from an expected voltage while sourcing power solely from said removable battery;
automatically and electronically command, by way of said microcontroller, said switching device to begin sourcing power solely from said removable battery following detection of a voltage increase at said voltage detection circuit from an expected voltage while sourcing power solely from said alternative power source and determination that a valid charge state exists at said removable battery; and
automatically and electronically command, by way of said microcontroller, said switching device to continue sourcing power solely from said alternative power source upon detection of said voltage increase at said voltage detection circuit from said expected voltage while sourcing power solely from said alternative power source and determination that said valid charge state does not exist at said removable battery.

2. The mobile medical cart of claim 1 wherein:
said connector is located at a lower surface of said holder;
said terminal is located at a lower surface of said removable battery;
said holder is located at a rear surface of said body;
said holder is configured to accommodate said removable battery in a snap-fit; and
said work platform comprises a flat surface and handles.

3. The mobile medical cart of any one of claims 1-2 further comprising:
a storage device (345) mounted to said body below said work platform; and
a tray (347) for a keyboard mounted to said body below said work platform, wherein:
a first one of said system boards is configured to control locking mechanisms, each provided at, and configured to selectively lock, one of a plurality of individual storage compartments within said storage device;
a second one of said system boards is configured to control one or more lights; and
a third one of said system boards is configured to control one or more motors for adjusting a height of said work platform or said monitor along said body.

4. The mobile medical cart of any one of claims 1-3 wherein:
said control system comprises a touch screen;
said control system is configured to display an indication of whether said switching devices is sourcing power from said removeable battery or said alternative power source;
said control system is configured to display an indication of a charge level of said removeable battery or said alternative power source; and
said control system is configured to generate an alert when the charge level of said removable battery or said alternative power source being sourced by said switching device falls below a predetermined threshold.

5. The mobile medical cart of any one of claims 1-4 further comprising:
an obstruction (114) configured to physically obstruct removal of the removable battery from the holder while said removable battery is located in a secured position within said holder until said removable battery is moved to an unsecured position; and
a detector positioned and configured to detect movement of said removable battery out of said secured position.

6. The mobile medical cart of claim 5 wherein:
said battery board is in electrical connection with the switching device and the detector and configured to, by way of said microcontroller and said switching device, automatically and electronically cease sourcing power from said removable battery and begin sourcing power from the alternative power source following receipt of one or more signals from said detector indicating that the removable battery is no longer in said secured position.

7. The mobile medical cart of any one of claims 5-6 wherein:
said holder comprises a basket configured for pivoting movement relative to a fixed portion of said mobile medical cart;
said detector comprises a switch (113) located between said basket and said fixed portion of said mobile medical cart;
said secured position comprises placement of the basket adjacent to the fixed portion of the mobile medical cart; and
said unsecured position comprises placement of the basket away from the fixed portion of the mobile medical cart.

8. The mobile medical cart of any one of claims 5-7 wherein:
said connector is configured for movement sufficient to maintain an electrical connection between said terminal and said connector while said removable battery is positioned within said holder and moved between said secured position and said unsecured position.

9. The mobile medical cart of any one of claims 5-8 further comprising:
a protrusion (117) located at said holder, wherein said holder is fixed to said body, said connector is configured for pivoting movement within said holder, and said detector is located within said holder; and
a cavity (119) defined by a housing of said removable battery and configured to accept said protrusion when said removable battery is placed within said holder in the secured position or the unsecured position to align said terminal with said connector.

10. The mobile medical cart of any one of claims 1-9 wherein:
the battery board is configured to derive the expected voltage while sourcing power solely from said removable battery from steady-state voltage supply during normal operations of said mobile medical cart while sourcing power solely from said removable battery; and
the battery board is configured to derive the expected voltage while sourcing power solely from said alternative power source from steady-state voltage supply during normal operations of said mobile medical cart while sourcing power solely from said alternative power source.

11. A method of providing data continuity for a mobile medical cart (310), said method
comprising the steps of:
providing the mobile medical cart (310) comprising a battery board (360) comprising a voltage detection circuit (362), a microcontroller (365), and a switching device (366), wherein said switching device is electrically interposed between an alternative power source (338) comprising a back-up battery configured to power said mobile medical cart for a period of time, a connector (152) of a holder (312) configured to removably receive a removeable battery (320) in an interchangeable fashion with one removable batteries (320), and system boards (368), each configured to control at least one function of said mobile medical cart;
electronically and automatically commanding, by way of said microcontroller, said switching device to begin sourcing power solely from said alternative power source following detection of a voltage decrease at said voltage detection circuit from an expected voltage when sourcing power solely from said removable battery;
electronically and automatically commanding, by way of said microcontroller, said switching device to begin sourcing power solely from said removable battery following detection of a voltage increase at said voltage detection circuit from an expected voltage when sourcing power solely from said alternative power source and a determination that a valid charge state exists at said removeable battery; and
electronically and automatically commanding, by way of said microcontroller, said switching device to continue sourcing power solely from said alternative power source following detection of said voltage increase at said voltage detection circuit from said expected voltage when sourcing power solely from said alternative power source and a determination that said valid charge state does not exist at said removable battery;
wherein said mobile medical cart comprises a base with wheels for moving said mobile medical cart about a ground surface and a body (346) extending vertically from said base;
wherein said holder is located at said body (346) above said base; and
wherein said back-up battery is permanently installed within said base.

12. The method of claim 11 wherein:
said mobile medical cart comprises:
a display (342) mounted to an upper portion of said body;
a work platform (344) mounted to said body between said display and said base and configured for sliding movement inward and outward relative to said body;
a connector (152) configured for selective electrical connection with a terminal of said removable battery;
a storage device (345) comprising drawers mounted to said body below said work platform;
a tray (347) for a keyboard mounted to said body below said work platform; and
a control system (343) including a touch screen for displaying icons related to said functions and receiving user input at said icons, wherein said control system is in electronic communication with said system boards for commanding changes to said functions of said mobile medical cart based upon said user input received at said touch screen;
said system boards are configured to control locking mechanisms for each of said drawers, lights, and one or more motors for adjusting a height of said work platform along said body relative to said ground surface;
said control system is located at said work platform;
said holder is mounted to a rear surface of said body; and
said work platform comprises a flat surface and handles.

13. The method of any one of claims 11-12 further comprising the steps of:
generating an alert when a charge level of said removeable battery or said alternative power source falls below a predetermined threshold;
electronically displaying an indication of whether said switching device is sourcing power from said removeable battery or said alternative power source; and
electronically displaying said charge level of said removeable battery and said charge level of said alternative power source.

14. The method of any one of claims 11-13 further comprising the steps of:
securing the removable battery within the holder, said holder being fixed and defining a cavity (119) configured to receive and temporarily secure said removable battery in a secured position, wherein an obstruction (114) is provided in association with said holder which physically prevents removal said removable battery from said holder until said removable battery is moved into an unsecured position where at least a portion of said removable battery is moved away from said holder;
determining, by way of a detector positioned within said cavity, that said removable battery is no longer in the secured position; and
electronically and automatically commanding, by way of said microcontroller, said switching device to cease sourcing power from said removable battery and begin sourcing power solely from the alternative power source following said determination that said removable battery is no longer in said secured position.

15. A mobile medical cart (310) providing data continuity, said mobile medical cart comprising:
a base (332) comprising wheels (334) for moving said mobile medical cart about a ground surface;
a body (346) extending upward from said base;
a display (342) mounted to an upper portion of said body;
system boards (368) for controlling functions of said mobile medical cart;
a work platform (344) mounted to said body between said display and said wheeled base;
a control system (343) in electronic communication with said system boards and configured to receive user input and command changes to said functions of said mobile medical cart based upon said user input;
a holder (312) mounted to the body and configured to removably receive a removable battery comprising a terminal;
a connector (152) located at the holder and positioned to receive the terminal when said removable battery is positioned within the holder;
an alternative power source (338);
an obstruction (114) configured to physically obstruct removal of the removable battery from the holder while said removable battery is located in a secured position within said holder until said removable battery is moved to an unsecured position; and
a detector positioned and configured to detect movement of said removable battery out of said secured position, wherein said holder comprises a basket configured for pivoting movement relative to a fixed portion of said mobile medical cart, said detector comprises a switch located between said basket and said fixed portion of said mobile medical cart, said secured position comprises placement of the basket adjacent to the fixed portion of the mobile medical cart, and said unsecured position comprises placement of the basket away from the fixed portion of the mobile medical cart; and
a battery board (360) comprising a voltage detection circuit, a microcontroller, and a switching device, wherein said switching device is electrically interposed between said alternative power source, said connector of said holder, and each of said system boards, wherein said battery board is configured to:
automatically and electronically command, by way of said microcontroller, said switching device to begin sourcing power solely from said alternative power source following detection of a voltage decrease at said voltage detection circuit from an expected voltage while sourcing power solely from said removable battery;
automatically and electronically command, by way of said microcontroller, said switching device to begin sourcing power solely from said removable battery following detection of a voltage increase at said voltage detection circuit from an expected voltage while sourcing power solely from said alternative power source and determination that a valid charge state exists at said removable battery; and
automatically and electronically command, by way of said microcontroller, said switching device to continue sourcing power solely from said alternative power source upon detection of said voltage increase at said voltage detection circuit from said expected voltage while sourcing power solely from said alternative power source and determination that said valid charge state does not exist at said removable battery.

## Patentansprüche

1. Mobiler medizinischer Wagen (310) mit Bereitstellung von Datenkontinuität, wobei der mobile medizinische Wagen umfasst:
eine Basis (332), die Räder (334) umfasst, um den mobilen medizinischen Wagen auf einer Bodenoberfläche zu bewegen;
einen Körper (346), der sich von der Basis nach oben erstreckt;
eine Anzeige (342), die an einem oberen Abschnitt des Körpers montiert ist;
Systemplatinen (368) zum Steuern von Funktionen des mobilen medizinischen Wagens;
eine Arbeitsplattform (344), die an dem Körper zwischen der Anzeige und dem fahrbaren Untersatz montiert ist;
ein Steuersystem (343) in elektronischer Kommunikation mit den Systemplatinen und konfiguriert, um Benutzereingaben und Befehlsänderungen an den Funktionen des mobilen medizinischen Wagens basierend auf der Benutzereingabe zu empfangen;
einen Halter (312), der an dem Körper oberhalb der Basis montiert und konfiguriert ist, um einen entfernbaren Akku (320), der einen Anschluss (115) umfasst, in einer austauschbaren Weise mit anderen entfernbaren Akkus (320) entfernbar aufzunehmen;
einen Verbinder (152), der sich an dem Halter befindet und positioniert ist, um den Anschluss aufzunehmen, wenn der entfernbare Akku innerhalb des Halters positioniert ist;
eine alternative Energiequelle (338), die eine Stützbatterie umfasst, die dauerhaft innerhalb des mobilen medizinischen Wagens installiert ist und konfiguriert ist, um den mobilen medizinischen Wagen für einen Zeitraum mit Energie zu versorgen; und
eine Batterieplatine (360), die eine Spannungserfassungsschaltung (362), einen Mikrocontroller (364) und eine Schaltvorrichtung (366) umfasst, wobei die Schaltvorrichtung zwischen der alternativen Energiequelle, dem Verbinder der Halterung und jeder der Systemplatinen elektrisch angeordnet ist, wobei die Batterieplatine konfiguriert ist zum:
automatischen und elektronischen Befehlen, über den Mikrocontroller, der Schaltvorrichtung, die Energieversorgung ausschließlich von der alternativen Energiequelle zu starten, nach dem Erfassen eines Spannungsabfalls an der Spannungserfassungsschaltung von einer erwarteten Spannung, während die Energieversorgung ausschließlich von dem entfernbaren Akku erfolgt;
automatischen und elektronischen Befehlen, über den Mikrocontroller, der Schaltvorrichtung, die Energieversorgung ausschließlich von dem entfernbaren Akku zu starten, nach dem Erfassen eines Spannungsanstiegs an der Spannungserfassungsschaltung von einer erwarteten Spannung, während die Energieversorgung ausschließlich von der alternativen Energiequelle erfolgt, und dem Bestimmen, dass ein gültiger Ladezustand an dem entfernbaren Akku vorhanden ist; und
automatischen und elektronischen Befehlen, über den Mikrocontroller, der Schaltvorrichtung, die Energieversorgung ausschließlich von dem entfernbaren Akku fortzusetzen, bei Erfassen des Spannungsanstiegs an der Spannungserfassungsschaltung von der erwarteten Spannung, während die Energieversorgung ausschließlich von der alternativen Energiequelle erfolgt und bei Bestimmen, dass der gültige Ladezustand an dem entfernbaren Akku nicht vorhanden ist.

2. Mobiler medizinischer Wagen nach Anspruch 1, wobei:
der Verbinder an einer unteren Oberfläche des Halters angeordnet ist;
der Anschluss sich an einer unteren Oberfläche des entfernbaren Akkus befindet;
der Halter sich an einer hinteren Oberfläche des Körpers befindet;
der Halter konfiguriert ist, um den entfernbaren Akku in einem Schnappverschluss aufzunehmen; und
die Arbeitsplattform eine flache Oberfläche und Griffe umfasst.

3. Mobiler medizinischer Wagen nach einem der Ansprüche 1 bis 2, ferner umfassend:
eine Aufbewahrungsvorrichtung (345), die an dem Körper unter der Arbeitsplattform montiert ist; und
eine Ablage (347) für eine Tastatur, die an dem Körper unterhalb der Arbeitsplattform montiert ist, wobei:
eine erste der Systemplatinen konfiguriert ist, um Verriegelungsmechanismen zu steuern, die jeweils an einem von einer Vielzahl von einzelnen Aufbewahrungsfächern innerhalb der Aufbewahrungsvorrichtung vorgesehen und konfiguriert sind, um dieses selektiv zu verriegeln;
eine zweite der Systemplatinen konfiguriert ist, um ein oder mehrere Leuchten zu steuern; und
eine dritte der Systemplatinen konfiguriert ist, um einen oder mehrere Motoren zum Einstellen einer Höhe der Arbeitsplattform oder des Monitors entlang des Körpers zu steuern.

4. Mobiler medizinischer Wagen nach einem der Ansprüche 1-3, wobei:
das Steuersystem einen Touchscreen umfasst;
das Steuersystem konfiguriert ist, um eine Angabe darüber anzuzeigen, ob die Schaltvorrichtungen Energie von dem entfernbaren Akku oder der alternativen Energiequelle beziehen;
das Steuersystem konfiguriert ist, um eine Angabe eines Ladezustands des entfernbaren Akkus oder der alternativen Energiequelle anzuzeigen; und
das Steuersystem konfiguriert ist, um eine Warnung zu erzeugen, wenn der Ladezustand des entfernbaren Akkus oder der alternativen Energiequelle, die von der Schaltvorrichtung gespeist wird, unter einen vorbestimmten Schwellenwert fällt.

5. Mobiler medizinischer Wagen nach einem der Ansprüche 1-4, ferner umfassend:
ein Hindernis (114), das konfiguriert ist, um das Entfernen des entfernbaren Akkus von der Halterung physisch zu behindern, während sich der entfernbare Akku in einer gesicherten Position innerhalb des Halters befindet, bis der entfernbare Akku in eine ungesicherte Position bewegt wird; und
einen Detektor, der positioniert und konfiguriert ist, um eine Bewegung des entfernbaren Akkus aus der gesicherten Position zu erfassen.

6. Mobiler medizinischer Wagen nach Anspruch 5, wobei:
die Batterieplatine in elektrischer Verbindung mit der Schaltvorrichtung und dem Detektor steht und konfiguriert ist, um über den Mikrocontroller und die Schaltvorrichtung, eine Energieversorgung von dem entfernbaren Akku automatisch und elektronisch einzustellen und eine Energieversorgung von der alternativen Energiequelle nach Empfang eines oder mehrerer Signale von dem Detektor zu beginnen, die angeben, dass sich der entfernbare Akku nicht mehr in der gesicherten Position befindet.

7. Mobiler medizinischer Wagen nach einem der Ansprüche 5 bis 6, wobei:
der Halter einen Korb umfasst, der für eine Schwenkbewegung relativ zu einem festen Abschnitt des mobilen medizinischen Wagens konfiguriert ist;
der Detektor einen Schalter (113) umfasst, der sich zwischen dem Korb und dem festen Abschnitt des mobilen medizinischen Wagens befindet;
die befestigte Position das Platzieren des Korbes angrenzend an den festen Abschnitt des mobilen medizinischen Wagens umfasst; und
die ungesicherte Position das Platzieren des Korbes entfernt von dem festen Abschnitt des mobilen medizinischen Wagens umfasst.

8. Mobiler medizinischer Wagen nach einem der Ansprüche 5 bis 7, wobei:
der Verbinder für eine Bewegung konfiguriert ist, die ausreicht, um eine elektrische Verbindung zwischen dem Anschluss und dem Verbinder aufrechtzuerhalten, während der entfernbare Akku innerhalb des Halters positioniert und zwischen der gesicherten Position und der ungesicherten Position bewegt wird.

9. Mobiler medizinischer Wagen nach einem der Ansprüche 5 bis 8, ferner umfassend:
einen Vorsprung (117), der sich an dem Halter befindet, wobei der Halter an dem Körper befestigt ist, wobei der Verbinder für eine Schwenkbewegung innerhalb des Halters konfiguriert ist und der Detektor sich innerhalb des Halters befindet; und
einen Hohlraum (119), der durch ein Gehäuse des entfernbaren Akkus definiert und konfiguriert ist, um den Vorsprung aufzunehmen, wenn der entfernbare Akku in dem Halter in der gesicherten Position oder der ungesicherten Position platziert ist, um den Anschluss mit dem Verbinder auszurichten.

10. Mobiler medizinischer Wagen nach einem der Ansprüche 1 bis 9, wobei:
die Batterieplatine konfiguriert ist, um die erwartete Spannung, während sie Energie ausschließlich von dem entfernbaren Akku bezieht, aus der stationären Spannungsversorgung während des normalen Betriebs des mobilen medizinischen Wagens abzuleiten, während sie Energie ausschließlich von dem entfernbaren Akku bezieht; und
die Batterieplatine konfiguriert ist, um die erwartete Spannung, während sie Energie ausschließlich von der alternativen Energiequelle bezieht, aus der stationären Spannungsversorgung während des normalen Betriebs des mobilen medizinischen Wagens abzuleiten, während sie Energie ausschließlich von der alternativen Energiequelle bezieht.

11. Verfahren zum Bereitstellen von Datenkontinuität für einen mobilen medizinischen Wagen (310), wobei das Verfahren die folgenden Schritte umfasst:
Bereitstellen des mobilen medizinischen Wagens (310), der eine Batterieplatine (360) umfasst, die eine Spannungserfassungsschaltung (362), einen Mikrocontroller (365) und eine Schaltvorrichtung (366) umfasst, wobei die Schaltvorrichtung elektrisch zwischen einer alternativen Energiequelle (338) angeordnet ist, die eine Stützbatterie umfasst, die so konfiguriert ist, dass sie den mobilen medizinischen Wagen für eine Zeitspanne mit Energie versorgt, einem Verbinder (152) eines Halters (312), der so konfiguriert ist, dass er einen entfernbaren Akku (320) in einer austauschbaren Weise mit einem entfernbaren Akkus (320) aufnehmen kann, und Systemplatinen (368), die jeweils so konfiguriert sind, dass sie mindestens eine Funktion des mobilen medizinischen Wagens steuern;
elektronisches und automatisches Befehlen, über den Mikrocontroller, der Schaltvorrichtung, die Energieversorgung ausschließlich von der alternativen Energiequelle zu starten, nach dem Erfassen eines Spannungsabfalls an der Spannungserfassungsschaltung von einer erwarteten Spannung, wenn die Energieversorgung ausschließlich von dem abnehmbaren Akku erfolgt;
elektronisches und automatisches Befehlen, über den Mikrocontroller, der Schaltvorrichtung, die Energieversorgung ausschließlich von dem entfernbaren Akku zu starten, nach dem Erfassen eines Spannungsanstiegs an der Spannungserfassungsschaltung von einer erwarteten Spannung, wenn die Energieversorgung ausschließlich von der alternativen Energiequelle erfolgt, und dem Bestimmen, dass ein gültiger Ladezustand an dem entfernbaren Akku vorhanden ist; und
elektronisches und automatisches Befehlen, über den Mikrocontroller, der Schaltvorrichtung, die Energieversorgung ausschließlich von dem entfernbaren Akku fortzusetzen, nach dem Erfassen des Spannungsanstiegs an der Spannungserfassungsschaltung von der erwarteten Spannung, wenn die Energieversorgung ausschließlich von der alternativen Energiequelle erfolgt und nach dem Bestimmen, dass der gültige Ladezustand an dem entfernbaren Akku nicht vorhanden ist;
wobei der mobile medizinische Wagen eine Basis mit Rädern zum Bewegen des mobilen medizinischen Wagens über eine Bodenoberfläche und einen Körper (346) umfasst, der sich vertikal von der Basis erstreckt;
wobei sich der Halter an dem Körper (346) oberhalb der Basis befindet; und
wobei die Stützbatterie dauerhaft innerhalb der Basis installiert ist.

12. Verfahren nach Anspruch 11, wobei:
der mobile medizinische Wagen umfasst:
eine Anzeige (342), die an einem oberen Abschnitt des Körpers montiert ist;
eine Arbeitsplattform (344), die an dem Körper zwischen der Anzeige und der Basis montiert und für eine Gleitbewegung nach innen und nach außen relativ zu dem Körper konfiguriert ist;
einen Verbinder (152), der für eine selektive elektrische Verbindung mit einem Anschluss des entfernbaren Akkus konfiguriert ist;
eine Aufbewahrungsvorrichtung (345), umfassend Schubladen, die an dem Körper unter der Arbeitsplattform montiert sind;
eine Ablage (347) für eine Tastatur, die an dem Körper unterhalb der Arbeitsplattform montiert ist; und ein Steuersystem (343), das einen Touchscreen zum Anzeigen von Symbolen einschließt, die sich auf die Funktionen beziehen, und zum Empfangen von Benutzereingaben an den Symbolen, wobei das Steuersystem in elektronischer Kommunikation mit den Systemplatinen steht, um Änderungen an den Funktionen des mobilen medizinischen Wagens basierend auf der Benutzereingabe zu befehlen, die an dem Touchscreen empfangen wird;
die Systemplatinen konfiguriert sind, um Verriegelungsmechanismen für jede der Schubladen, Leuchten und einen oder mehrere Motoren zum Einstellen einer Höhe der Arbeitsplattform entlang des Körpers relativ zu der Bodenoberfläche zu steuern;
das Steuersystem sich an der Arbeitsplattform befindet;
der Halter an einer hinteren Oberfläche des Körpers montiert ist; und
die Arbeitsplattform eine flache Oberfläche und Griffe umfasst.

13. Verfahren nach einem der Ansprüche 11 bis 12, ferner umfassend die Schritte:
Erzeugen einer Warnung, wenn ein Ladezustand des entfernbaren Akkus oder der alternativen Energiequelle unter einen vorbestimmten Schwellenwert fällt;
elektronisches Anzeigen einer Angabe, ob die Schaltvorrichtung eine Energie von dem entfernbaren Akku oder der alternativen Energiequelle bezieht; und
elektronisches Anzeigen des Ladezustands des entfernbaren Akkus und des Ladezustands der alternativen Energiequelle.

14. Verfahren nach einem der Ansprüche 11 bis 13, ferner umfassend die Schritte:
Befestigen des entfernbaren Akkus in dem Halter, wobei der Halter fixiert ist und einen Hohlraum (119) definiert, der so konfiguriert ist, dass er den entfernbaren Akku in einer gesicherten Position aufnimmt und vorübergehend sichert, wobei ein Hindernis (114) in Verbindung mit dem Halter vorgesehen ist, das physisch verhindert, dass der entfernbare Akku aus dem Halter entfernt wird, bis der entfernbare Akku in eine ungesicherte Position bewegt wird, in der mindestens ein Teil des entfernbaren Akkus von dem Halter weg bewegt wird;
Bestimmen, durch einen Detektor, der innerhalb des Hohlraums positioniert ist, dass sich der entfernbare Akku nicht mehr in der gesicherten Position befindet; und
elektronisches und automatisches Befehlen, über den Mikrocontroller, der Schaltvorrichtung, die Energieversorgung von dem entfernbaren Akku zu beenden und die Energieversorgung ausschließlich von der alternativen Energiequelle zu beginnen, nachdem festgestellt wurde, dass sich der entfernbare Akku nicht mehr in der gesicherten Position befindet.

15. Mobiler medizinischer Wagen (310) mit Bereitstellung von Datenkontinuität, wobei der mobile medizinische Wagen umfasst:
eine Basis (332), die Räder (334) umfasst, um den mobilen medizinischen Wagen auf einer Bodenoberfläche zu bewegen;
einen Körper (346), der sich von der Basis nach oben erstreckt;
eine Anzeige (342), die an einem oberen Abschnitt des Körpers montiert ist;
Systemplatinen (368) zum Steuern von Funktionen des mobilen medizinischen Wagens;
eine Arbeitsplattform (344), die an dem Körper zwischen der Anzeige und dem fahrbaren Untersatz montiert ist;
ein Steuersystem (343) in elektronischer Kommunikation mit den Systemplatinen und konfiguriert, um Benutzereingaben und Befehlsänderungen an den Funktionen des mobilen medizinischen Wagens basierend auf der Benutzereingabe zu empfangen;
einen Halter (312), der an dem Körper montiert und konfiguriert ist, um einen entfernbaren Akku, der einen Anschluss umfasst, entfernbar aufzunehmen;
einen Verbinder (152), der sich an dem Halter befindet und positioniert ist, um den Anschluss aufzunehmen, wenn der entfernbare Akku innerhalb des Halters positioniert ist;
eine alternative Leistungsquelle (338);
ein Hindernis (114), das konfiguriert ist, um das Entfernen des entfernbaren Akkus von der Halterung physisch zu behindern, während sich der entfernbare Akku in einer gesicherten Position innerhalb des Halters befindet, bis der entfernbare Akku in eine ungesicherte Position bewegt wird; und
einen Detektor, der so positioniert und konfiguriert ist, dass er eine Bewegung des entfernbaren Akkus aus der gesicherten Position heraus erfasst, wobei der Halter einen Korb umfasst, der für eine Schwenkbewegung relativ zu einem festen Abschnitt des mobilen medizinischen Wagens konfiguriert ist, wobei der Detektor einen Schalter umfasst, der zwischen dem Korb und dem festen Abschnitt des mobilen medizinischen Wagens angeordnet ist, wobei die gesicherte Position eine Platzierung des Korbs angrenzend an den festen Abschnitt des mobilen medizinischen Wagens umfasst, und wobei die ungesicherte Position eine Platzierung des Korbs weg von dem festen Abschnitt des mobilen medizinischen Wagens umfasst; und
eine Batterieplatine (360), die eine Spannungserfassungsschaltung, einen Mikrocontroller und eine Schaltvorrichtung umfasst, wobei die Schaltvorrichtung zwischen der alternativen Energiequelle, dem Verbinder der Halterung und jeder der Systemplatinen elektrisch angeordnet ist, wobei die Batterieplatine konfiguriert ist zum:
automatisches und elektronisches Befehlen, über den Mikrocontroller, der Schaltvorrichtung, die Energieversorgung ausschließlich von der alternativen Energiequelle zu starten, nach dem Erfassen eines Spannungsabfalls an der Spannungserfassungsschaltung von einer erwarteten Spannung, während die Energieversorgung ausschließlich von dem entfernbaren Akku erfolgt;
automatisches und elektronisches Befehlen, über den Mikrocontroller, der Schaltvorrichtung, die Energieversorgung ausschließlich von dem entfernbaren Akku zu starten, nach dem Erfassen eines Spannungsanstiegs an der Spannungserfassungsschaltung von einer erwarteten Spannung, während die Energieversorgung ausschließlich von der alternativen Energiequelle erfolgt, und dem Bestimmen, dass ein gültiger Ladezustand an dem entfernbaren Akku vorhanden ist; und
automatisches und elektronisches Befehlen, über den Mikrocontroller, der Schaltvorrichtung, die Energieversorgung ausschließlich von dem entfernbaren Akku fortzusetzen, bei Erfassen des Spannungsanstiegs an der Spannungserfassungsschaltung von der erwarteten Spannung, während die Energieversorgung ausschließlich von der alternativen Energiequelle erfolgt und bei Bestimmen, dass der gültige Ladezustand an dem entfernbaren Akku nicht vorhanden ist.

## Revendications

1. Chariot médical mobile (310) fournissant une continuité de données, ledit chariot médical mobile comprenant :
une base (332) comprenant des roues (334) pour le déplacement dudit chariot médical mobile sur une surface de sol ;
un corps (346) s'étendant vers le haut à partir de ladite base ;
un affichage (342) monté sur une partie supérieure dudit corps ;
des cartes systèmes (368) pour la commande de fonctions dudit chariot médical mobile ;
une plate-forme de travail (344) montée sur ledit corps entre ledit affichage et ladite base à roues ;
un système de commande (343) en communication électronique avec lesdites cartes systèmes et configuré pour recevoir une entrée utilisateur et commander des changements desdites fonctions dudit chariot médical mobile sur la base de ladite entrée utilisateur ;
un support (312) monté sur le corps au-dessus de ladite base et conçu pour recevoir de manière amovible une batterie amovible (320) comprenant une borne (115) d'une manière interchangeable avec d'autres batteries amovibles (320) ;
un connecteur (152) situé au niveau du support et positionné pour recevoir la borne lorsque ladite batterie amovible est positionnée à l'intérieur du support ;
une source d'énergie alternative (338) comprenant une batterie de secours installée en permanence à l'intérieur dudit chariot médical mobile et configurée pour approvisionner ledit chariot médical mobile en énergie pendant une période de temps ; et
une carte de batterie (360) comprenant un circuit de détection de tension (362), un microcontrôleur (364), et un dispositif de commutation (366), dans lequel ledit dispositif de commutation est électriquement interposé entre ladite source d'énergie alternative, ledit connecteur dudit support, et chacune desdites cartes systèmes, dans lequel ladite carte de batterie est configurée pour :
commander automatiquement et électroniquement, au moyen dudit microcontrôleur, ledit dispositif de commutation pour commencer l'approvisionnement en énergie uniquement à partir de ladite source d'énergie alternative suite à la détection d'une diminution de tension au niveau dudit circuit de détection de tension à partir d'une tension attendue pendant l'approvisionnement en énergie uniquement à partir de ladite batterie amovible ;
commander automatiquement et électroniquement, au moyen dudit microcontrôleur, ledit dispositif de commutation pour commencer l'approvisionnement en énergie uniquement à partir de ladite batterie amovible suite à la détection d'une augmentation de tension au niveau dudit circuit de détection de tension à partir d'une tension attendue pendant l'approvisionnement en énergie uniquement à partir de ladite source d'énergie alternative et de la détermination selon laquelle un état de charge valide existe au niveau de ladite batterie amovible ; et
commander automatiquement et électroniquement, au moyen dudit microcontrôleur, ledit dispositif de commutation pour continuer l'approvisionnement en énergie uniquement à partir de ladite source d'énergie alternative lors de la détection de ladite augmentation de tension au niveau dudit circuit de détection de tension à partir de ladite tension attendue pendant l'approvisionnement en énergie uniquement à partir de ladite source d'énergie alternative et de la détermination selon laquelle ledit état de charge valide n'existe pas au niveau de ladite batterie amovible.

2. Chariot médical mobile selon la revendication 1 dans lequel :
ledit connecteur est situé au niveau d'une surface inférieure dudit support ;
ladite borne est située au niveau d'une surface inférieure de ladite batterie amovible ;
ledit support est situé au niveau d'une surface arrière dudit corps ;
ledit support est conçu pour accueillir ladite batterie amovible dans un encliquetage ; et
ladite plate-forme de travail comprend une surface plane et des poignées.

3. Chariot médical mobile selon l'une quelconque des revendications 1 et 2 comprenant en outre :
un dispositif de stockage (345) monté sur ledit corps en dessous de ladite plate-forme de travail ; et
un plateau (347) pour un clavier monté sur ledit corps en dessous de ladite plate-forme de travail, dans lequel :
une première carte système desdites cartes systèmes est configurée pour commander des mécanismes de verrouillage, chacun prévu au niveau de, et configuré pour, verrouiller sélectivement, un compartiment de stockage individuel d'une pluralité de compartiments de stockage individuels à l'intérieur dudit dispositif de stockage ;
une deuxième carte système desdites cartes systèmes est configurée pour commander une ou plusieurs lumières ; et
une troisième carte système desdites cartes systèmes est configurée pour commander un ou plusieurs moteurs pour le réglage d'une hauteur de ladite plate-forme de travail ou dudit moniteur le long dudit corps.

4. Chariot médical mobile selon l'une quelconque des revendications 1 à 3 dans lequel: ledit système de commande comprend un écran tactile ;
ledit système de commande est configuré pour afficher une indication indiquant si lesdits dispositifs de commutation est en train de s'approvisionner en énergie à partir de ladite batterie amovible ou de ladite source d'énergie alternative ;
ledit système de commande est configuré pour afficher une indication d'un niveau de charge de ladite batterie amovible ou de ladite source d'énergie alternative ; et
ledit système de commande est configuré pour générer une alerte lorsque le niveau de charge de ladite batterie amovible ou de ladite source d'énergie alternative à partir de laquelle ledit dispositif de commutation est en train de s'approvisionner tombe en dessous d'un seuil prédéterminé.

5. Chariot médical mobile selon l'une quelconque des revendications 1 à 4 comprenant en outre :
un moyen d'obstruction (114) conçu pour obstruer physiquement le retrait de la batterie amovible du support pendant que ladite batterie amovible est située dans une position immobilisée à l'intérieur dudit support jusqu'à ce que ladite batterie amovible soit déplacée vers une position non immobilisée ; et
un détecteur positionné et configuré pour détecter un mouvement de ladite batterie amovible hors de ladite position immobilisée.

6. Chariot médical mobile selon la revendication 5 dans lequel :
ladite carte de batterie est en connexion électrique avec le dispositif de commutation et le détecteur et est configurée pour, au moyen dudit microcontrôleur et dudit dispositif de commutation, arrêter automatiquement et électroniquement l'approvisionnement en énergie à partir de ladite batterie amovible et commencer l'approvisionnement en énergie à partir de la source d'énergie alternative suite à la réception d'un ou de plusieurs signaux provenant dudit détecteur indiquant que la batterie amovible n'est plus dans ladite position immobilisée.

7. Chariot médical mobile selon l'une quelconque des revendications 5 à 6 dans lequel :
ledit support comprend un panier conçu pour un mouvement de pivotement par rapport à une partie fixe dudit chariot médical mobile ;
ledit détecteur comprend un commutateur (113) situé entre ledit panier et ladite partie fixe dudit chariot médical mobile ;
ladite position immobilisée comprend la mise en place du panier de manière adjacente à la partie fixe du chariot médical mobile ; et
ladite position non immobilisée comprend la mise en place du panier à l'écart de la partie fixe du chariot médical mobile.

8. Chariot médical mobile selon l'une quelconque des revendications 5 à 7 dans lequel :
ledit connecteur est conçu pour un mouvement suffisant pour maintenir une connexion électrique entre ladite borne et ledit connecteur pendant que ladite batterie amovible est positionnée à l'intérieur dudit support et déplacée entre ladite position immobilisée et ladite position non immobilisée.

9. Chariot médical mobile selon l'une quelconque des revendications 5 à 8 comprenant en outre :
une saillie (117) située au niveau dudit support, dans lequel ledit support est fixé audit corps, ledit connecteur est conçu pour un mouvement de pivotement à l'intérieur dudit support, et ledit détecteur est situé à l'intérieur dudit support ; et
une cavité (119) définie par un boîtier de ladite batterie amovible et conçue pour accepter ladite saillie lorsque ladite batterie amovible est placée à l'intérieur dudit support dans la position immobilisée ou la position non immobilisée pour aligner ladite borne sur ledit connecteur.

10. Chariot médical mobile selon l'une quelconque des revendications 1 à 9 dans lequel :
la carte de batterie est configurée pour déduire la tension attendue pendant l'approvisionnement en énergie uniquement à partir de ladite batterie amovible à partir d'un approvisionnement en tension en régime permanent pendant des fonctionnements normaux dudit chariot médical mobile pendant l'approvisionnement en énergie uniquement à partir de ladite batterie amovible ; et
la carte de batterie est configurée pour déduire la tension attendue pendant l'approvisionnement en énergie uniquement à partir de ladite source d'énergie alternative à partir d'un approvisionnement en tension en régime permanent pendant des fonctionnements normaux dudit chariot médical mobile pendant l'approvisionnement en énergie uniquement à partir de ladite source d'énergie alternative.

11. Procédé de fourniture d'une continuité de données pour un chariot médical mobile (310), ledit procédé comprenant les étapes consistant à :
fournir le chariot médical mobile (310) comprenant une carte de batterie (360) comprenant un circuit de détection de tension (362), un microcontrôleur (365), et un dispositif de commutation (366), dans lequel ledit dispositif de commutation est électriquement interposé entre une source d'énergie alternative (338) comprenant une batterie de secours configurée pour approvisionner ledit chariot médical mobile en énergie pendant une période de temps, un connecteur (152) d'un support (312) conçu pour recevoir de manière amovible une batterie amovible (320) d'une manière interchangeable avec une batteries amovibles (320), et des cartes systèmes (368), chacune configurée pour commander au moins une fonction dudit chariot médical mobile ;
commander électroniquement et automatiquement, au moyen dudit microcontrôleur, ledit dispositif de commutation pour commencer l'approvisionnement en énergie uniquement à partir de ladite source d'énergie alternative suite à la détection d'une diminution de tension au niveau dudit circuit de détection de tension à partir d'une tension attendue lors de l'approvisionnement en énergie uniquement à partir de ladite batterie amovible ;
commander électroniquement et automatiquement, au moyen dudit microcontrôleur, ledit dispositif de commutation pour commencer l'approvisionnement en énergie uniquement à partir de ladite batterie amovible suite à la détection d'une augmentation de tension au niveau dudit circuit de détection de tension à partir d'une tension attendue lors de l'approvisionnement en énergie uniquement à partir de ladite source d'énergie alternative et de la détermination selon laquelle un état de charge valide existe au niveau de ladite batterie amovible ; et
commander électroniquement et automatiquement, au moyen dudit microcontrôleur, ledit dispositif de commutation pour continuer l'approvisionnement en énergie uniquement à partir de ladite source d'énergie alternative suite à la détection de ladite augmentation de tension au niveau dudit circuit de détection de tension à partir de ladite tension attendue lors de l'approvisionnement en énergie uniquement à partir de ladite source d'énergie alternative et d'une détermination selon laquelle ledit état de charge valide n'existe pas au niveau de ladite batterie amovible ;
dans lequel ledit chariot médical mobile comprend une base avec des roues pour le déplacement dudit chariot médical mobile sur une surface de sol et un corps (346) s'étendant verticalement à partir de ladite base ;
dans lequel ledit support est situé au niveau dudit corps (346) au-dessus de ladite base ; et
dans lequel ladite batterie de secours est installée en permanence à l'intérieur de ladite base.

12. Procédé selon la revendication 11 dans lequel :
ledit chariot médical mobile comprend :
un affichage (342) monté sur une partie supérieure dudit corps ;
une plate-forme de travail (344) montée sur ledit corps entre ledit affichage et ladite base et conçue pour un mouvement coulissant vers l'intérieur et vers l'extérieur par rapport audit corps ;
un connecteur (152) configuré pour une connexion électrique sélective avec une borne de ladite batterie amovible ;
un dispositif de stockage (345) comprenant des tiroirs monté sur ledit corps en dessous de ladite plate-forme de travail ;
un plateau (347) pour un clavier monté sur ledit corps en dessous de ladite plate-forme de travail ; et un système de commande (343) comportant un écran tactile pour l'affichage d'icônes se rapportant auxdites fonctions et la réception d'une entrée utilisateur au niveau desdites icônes, dans lequel ledit système de commande est en communication électronique avec lesdites cartes systèmes pour la commande de changements desdites fonctions dudit chariot médical mobile sur la base de ladite entrée utilisateur reçue au niveau dudit écran tactile ;
lesdites cartes systèmes sont configurées pour commander des mécanismes de verrouillage pour chacun desdits tiroirs, des lumières, et un ou plusieurs moteurs pour le réglage d'une hauteur de ladite plate-forme de travail le long dudit corps par rapport à ladite surface de sol ;
ledit système de commande est situé au niveau de ladite plate-forme de travail ;
ledit support est monté sur une surface arrière dudit corps ; et
ladite plate-forme de travail comprend une surface plane et des poignées.

13. Procédé selon l'une quelconque des revendications 11 à 12 comprenant en outre les étapes consistant à :
générer une alerte lorsqu'un niveau de charge de ladite batterie amovible ou de ladite source d'énergie alternative tombe en dessous d'un seuil prédéterminé ;
afficher électroniquement une indication selon laquelle ledit dispositif de commutation est en train de s'approvisionner en énergie à partir de ladite batterie amovible ou de ladite source d'énergie alternative ; et
afficher électroniquement ledit niveau de charge de ladite batterie amovible et ledit niveau de charge de ladite source d'énergie alternative.

14. Procédé selon l'une quelconque des revendications 11 à 13 comprenant en outre les étapes consistant à :
immobiliser la batterie amovible à l'intérieur du support, ledit support étant fixe et définissant une cavité (119) conçue pour recevoir et immobiliser temporairement ladite batterie amovible dans une position immobilisée, dans lequel un moyen d'obstruction (114) est fourni en association avec ledit support qui empêche physiquement le retrait de ladite batterie amovible dudit support jusqu'à ce que ladite batterie amovible soit déplacée dans une position non immobilisée où au moins une partie de ladite batterie amovible est déplacée à l'écart dudit support ;
déterminer, au moyen d'un détecteur positionné à l'intérieur de ladite cavité, que ladite batterie amovible n'est plus dans la position immobilisée ; et
commander électroniquement et automatiquement, au moyen dudit microcontrôleur, ledit dispositif de commutation pour arrêter l'approvisionnement en énergie à partir de ladite batterie amovible et commencer l'approvisionnement en énergie uniquement à partir de la source d'énergie alternative suite à ladite détermination selon laquelle ladite batterie amovible n'est plus dans ladite position immobilisée.

15. Chariot médical mobile (310) fournissant une continuité de données, ledit chariot médical mobile comprenant :
une base (332) comprenant des roues (334) pour le déplacement dudit chariot médical mobile sur une surface de sol ;
un corps (346) s'étendant vers le haut à partir de ladite base ;
un affichage (342) monté sur une partie supérieure dudit corps ;
des cartes systèmes (368) pour la commande de fonctions dudit chariot médical mobile ;
une plate-forme de travail (344) montée sur ledit corps entre ledit affichage et ladite base à roues ;
un système de commande (343) en communication électronique avec lesdites cartes systèmes et configuré pour recevoir une entrée utilisateur et commander des changements desdites fonctions dudit chariot médical mobile sur la base de ladite entrée utilisateur ;
un support (312) monté sur le corps et conçu pour recevoir de manière amovible une batterie amovible comprenant une borne ;
un connecteur (152) situé au niveau du support et positionné pour recevoir la borne lorsque ladite batterie amovible est positionnée à l'intérieur du support ;
une source d'énergie alternative (338) ;
un moyen d'obstruction (114) conçu pour obstruer physiquement le retrait de la batterie amovible du support pendant que ladite batterie amovible est située dans une position immobilisée à l'intérieur dudit support jusqu'à ce que ladite batterie amovible soit déplacée vers une position non immobilisée ; et
un détecteur positionné et configuré pour détecter un mouvement de ladite batterie amovible hors de ladite position immobilisée, dans lequel ledit support comprend un panier conçu pour un mouvement de pivotement par rapport à une partie fixe dudit chariot médical mobile, ledit détecteur comprend un commutateur situé entre ledit panier et ladite partie fixe dudit chariot médical mobile, ladite position immobilisée comprend la mise en place du panier de manière adjacente à la partie fixe du chariot médical mobile, et ladite position non immobilisée comprend la mise en place du panier à l'écart de la partie fixe du chariot médical mobile ; et
une carte de batterie (360) comprenant un circuit de détection de tension, un microcontrôleur, et un dispositif de commutation, dans lequel ledit dispositif de commutation est électriquement interposé entre ladite source d'énergie alternative, ledit connecteur dudit support, et chacune desdites cartes systèmes, dans lequel ladite carte de batterie est configurée pour :
commander automatiquement et électroniquement, au moyen dudit microcontrôleur, ledit dispositif de commutation pour commencer l'approvisionnement en énergie uniquement à partir de ladite source d'énergie alternative suite à la détection d'une diminution de tension au niveau dudit circuit de détection de tension à partir d'une tension attendue pendant l'approvisionnement en énergie uniquement à partir de ladite batterie amovible ;
commander automatiquement et électroniquement, au moyen dudit microcontrôleur, ledit dispositif de commutation pour commencer l'approvisionnement en énergie uniquement à partir de ladite batterie amovible suite à la détection d'une augmentation de tension au niveau dudit circuit de détection de tension à partir d'une tension attendue pendant l'approvisionnement en énergie uniquement à partir de ladite source d'énergie alternative et de la détermination selon laquelle un état de charge valide existe au niveau de ladite batterie amovible ; et
commander automatiquement et électroniquement, au moyen dudit microcontrôleur, ledit dispositif de commutation pour continuer l'approvisionnement en énergie uniquement à partir de ladite source d'énergie alternative lors de la détection de ladite augmentation de tension au niveau dudit circuit de détection de tension à partir de ladite tension attendue pendant l'approvisionnement en énergie uniquement à partir de ladite source d'énergie alternative et de la détermination selon laquelle ledit état de charge valide n'existe pas au niveau de ladite batterie amovible.
